# FASCICULE DE BREVET EUROPEEN

(11) **EP 4 056 999 B1**
(45) Date de publication et mention de la délivrance du brevet: **30.08.2023**
(21) Numéro de dépôt: 22161004.1
(22) Date de dépôt: 09.03.2022
(51) Int. Cl.: G01N 33/24, G01N 23/223

(54) **PROCÉDÉ D'IDENTIFICATION DE LA COUCHE LITHOLOGIQUE D'UN MATÉRIAU EXCAVE EN VUE DE SA VALORISATION**
VERFAHREN ZUR IDENTIFIZIERUNG DER GESTEINSSCHICHT EINES AUSGEHOBENEN MATERIALS IN HINBLICK AUF SEINE VERWERTUNG
METHOD FOR IDENTIFYING THE LITHOLOGICAL LAYER OF AN EXCAVATED MATERIAL IN ORDER TO MAKE USE OF IT

(30) Priorité: 10.03.2021 FR 2102346
(43) Date de publication de la demande: 14.09.2022
(73) Titulaire: Eiffage GC Infra Linéaires, 78140 Vélizy-Villacoublay (FR)
(72) Inventeur: BOULANGÉ, Laurence, 38530 CHAPAREILLAN (FR); DOOM, Florian, 77144 MONTÉVRAIN (FR); JEANNELLE, Matthieu, 52190 SAINT BROINGT LES FOSSES (FR)
(74) Mandataire: Plasseraud IP

(56) Documents cités:
- WO-A1-2020/199292
- FR-A1- 3 062 917
- ROSS P. -S. ET AL: "Improving lithological discrimination in exploration drill-cores using portable X-ray fluorescence measurements: (1) testing three Olympus Innov-X analysers on unprepared cores", GEOCHEMISTRY: EXPLORATION, ENVIRONMENT, ANALYSIS, vol. 14, no. 2, 4 février 2014 (2014-02-04), pages 171-185, XP055853497, ISSN: 1467-7873, DOI: 10.1144/geochem2012-163

## Description

### Domaine technique

La présente divulgation relève du domaine de la valorisation des matériaux excavés en tant que matériau de construction, en particulier en tant que remblai ou granulat.

### Contexte de l'invention

Lors de grands travaux en génie civil, notamment lors de creusement de tunnels, une quantité très importante de matériaux excavés est générée en très peu de temps, ce qui nécessite une forte emprise sur les chantiers. Par exemple, un tunnelier utilisé pour la construction d'une ligne de métro extrait environ 800 tonnes par jour de matériaux excavés.

Classiquement les matériaux excavés sont stockés dans des installations de stockage de déchets ayant une emprise au sol très importante. Pour éviter ce problème, une solution consiste à valoriser ces matériaux excavés en nouveaux matériaux de construction.

Les matériaux excavés contiennent de nombreux éléments chimiques inorganiques différents qui, dans le temps, peuvent dégrader les propriétés de ces nouveaux matériaux de construction et altérer les ouvrages dans lesquels ils sont mis en oeuvre. C'est pourquoi il est nécessaire de déterminer la concentration massique en éléments chimiques inorganiques dans un matériau excavé avant de décider si et comment le valoriser.

Cependant déterminer la concentration massique en éléments chimiques inorganiques n'est pas suffisant. En effet deux matériaux excavés peuvent avoir la même composition en éléments chimiques inorganiques mais présenter des propriétés géomécaniques et chimiques différentes selon leur couche lithologique. C'est pourquoi il est également nécessaire d'identifier la couche lithologique du matériau excavé avant de décider si et comment le valoriser.

Actuellement la couche lithologique d'un matériau excavé est déterminée par analyse par diffraction aux rayons X. Cette méthode d'analyse permet de définir la structure cristalline de chaque phase cristalline constituant le matériau excavé pour ensuite identifier la couche lithologique du matériau excavé. Cependant cette méthode d'analyse est réalisée en plusieurs jours et requière une grande expertise pour interpréter les diffractogrammes. Elle nécessite également de préparer les échantillons à analyser par broyage du matériau excavé puis tamisage. Cette préparation est compliquée lorsque le matériau excavé est une roche très dure. De plus, cette méthode d'analyse requière des équipements sensibles et complexes à manipuler qui ne peuvent être situés directement sur le chantier car ils sont incompatibles avec les activités d'un chantier (vibration, poussière). Le laboratoire comprenant ces équipements sensibles est donc généralement situé loin du chantier. Cette méthode d'analyse est donc longue et compliquée à réaliser. De plus, durant cette longue période d'analyse, il est nécessaire de stocker les matériaux excavés sur les chantiers qui se trouvent très souvent implantés dans des zones géographiques à très fortes contraintes (zone urbaine, vallée montagneuse).

Le document Ross P.-S. et al.: "Improving lithological discrimination in exploration drill-cores using portable X-ray fluorescence measurements: (1) testing three Olympus Innov-X analysers on unprepared cores", Geochemistry Exploration Environment Analysis, Mai 2014, divulgue un procédé d'analyse d'un matériau excavé comprenant les étapes suivantes : a) analyse par spectroscopie par fluorescence aux rayons X d'un échantillon du matériau excavé pour déterminer la concentration massique de chacun des éléments chimiques inorganiques contenus dans ledit échantillon, l'étape a) étant réalisé sur n surfaces différentes de l'échantillon, et b) calcul de la moyenne des n concentrations massiques de chacun des éléments chimiques inorganiques contenus dans ledit échantillon et de l'écart type de cette moyenne.

Il existe encore un besoin d'un procédé plus simple et plus rapide pour déterminer la couche lithologique d'un matériau excavé pour déterminer si et comment un matériau excavé peut être valorisé pour optimiser les emprises de chantier, pour éviter les zones de stockage sur le chantier et pour permettre la revalorisation sûre d'une quantité importante de matériaux excavés.

De façon surprenante, la Demanderesse a trouvé un procédé d'analyse d'un matériau excavé permettant de répondre à ce besoin.

### Résumé

Selon un premier aspect, la présente divulgation porte sur un procédé d'identification de la couche lithologique d'un matériau excavé comprenant les étapes suivantes :
a) analyse par spectroscopie par fluorescence aux rayons X d'un échantillon du matériau excavé pour déterminer la concentration massique de chacun des éléments chimiques inorganiques contenus dans ledit échantillon,
   l'étape a) étant réalisé sur *n* surfaces différentes de l'échantillon pour déterminer *n* concentrations massiques de chacun des éléments chimiques inorganiques contenus dans ledit échantillon, avec *n* un nombre entier supérieur ou égal à 2, en particulier compris entre 3 et 20, tout particulièrement entre 5 et 15, encore plus particulièrement égal à 10,
b) calcul de la moyenne des *n* concentrations massiques de chacun des éléments chimiques inorganiques contenus dans ledit échantillon et de l'écart type de cette moyenne,
c) classification de chacun des éléments chimiques inorganiques contenus dans le matériau excavé selon les critères suivants :
   - l'éléments chimique inorganique dont la moyenne des *n* concentrations massiques dans le matériau excavé est supérieure à 1% et dont l'écart type de cette moyenne est inférieur à 40% est classé comme élément chimique inorganique majeur homogène,
   - l'éléments chimique inorganique dont la moyenne des *n* concentrations massiques dans le matériau excavé est supérieure à 1% et dont l'écart type de cette moyenne est supérieur à 40% est classé comme élément chimique inorganique majeur dispersé,
   - l'éléments chimique inorganique dont la moyenne des *n* concentrations massiques dans le matériau excavé est comprise entre 0,01 % et 1 % et dont l'écart type de cette moyenne est inférieur à 40% est classé comme élément chimique inorganique mineur homogène,
   - l'éléments chimique inorganique dont la moyenne des *n* concentrations massiques dans le matériau excavé est comprise entre 0,01 % et 1% et dont l'écart type de cette moyenne est supérieur à 40% est classé comme élément chimique inorganique mineur dispersé,
   - l'éléments chimique inorganique dont la moyenne des *n* concentrations massiques dans le matériau excavé est inférieure à 0,01% et dont l'écart type de cette moyenne est inférieure à 40% est classé comme élément chimique inorganique trace homogène, ou
   - l'éléments chimique inorganique dont la moyenne des *n* concentrations massiques dans le matériau excavé est inférieure à 0,01% et dont l'écart type de cette moyenne est supérieure à 40% est classé comme élément chimique inorganique trace dispersé, et
d) identification de la couche lithologique du matériau excavé à partir de la classification réalisée au cours de l'étape c).

L'étape a) d'analyse est avantageusement très rapide car elle peut être réalisée en quelques minutes.

L'étape a) est, en outre, très simple à réaliser. En particulier elle ne nécessite pas d'étape de préparation de l'échantillon du matériau excavé à analyser.

Cette étape a) d'analyse peut également être réalisée directement sur le chantier dans un laboratoire mobile comprenant un spectromètre à fluorescence X portable ou un spectromètre à fluorescence X facilement transportable car de taille réduite.

La moyenne des *n* concentrations massiques calculée par le procédé d'analyse de l'invention permet de classer de façon fiable les éléments chimiques inorganiques du matériau excavé parmi les éléments chimiques inorganiques majeurs, les éléments chimiques inorganiques mineurs et les éléments chimiques inorganiques traces du matériau excavé.

L'écart type de cette moyenne permet de classer de façon fiable les éléments chimiques inorganiques du matériau excavé en fonction de leur dispersibilité.

Sans vouloir être liés par aucune théorie; les inventeurs sont d'avis que le classement des éléments chimiques inorganiques du matériau excavé en éléments chimiques inorganiques majeurs homogènes ou dispersés, en éléments chimiques inorganiques mineurs homogènes ou dispersés et en éléments chimiques inorganiques traces homogènes ou dispersés grâce au procédé d'analyse de l'invention permet d'identifier la couche lithologique du matériau excavé de façon fiable, rapide et simple. Ainsi, de façon avantageuse, le procédé d'analyse de l'invention permet d'identifier ultérieurement la couche lithologique d'un matériau excavé puis, en fonction de cette identification, de décider si et comment le valoriser.

Ainsi, selon un autre aspect, la présente divulgation porte également sur un procédé de valorisation d'un matériau excavé mettant en oeuvre le procédé d'identification selon l'invention, comprenant en outre l'étape suivante : e) valorisation du matériau excavé en tant que matériau de construction en fonction de l'identification obtenue lors de l'étape d).

### Description des modes de réalisation

Selon un premier aspect, la présente divulgation porte sur un procédé d'identification de la couche lithologique d'un matériau excavé comprenant les étapes suivantes :
a) analyse par spectroscopie par fluorescence aux rayons X d'un échantillon du matériau excavé pour déterminer la concentration massique de chacun des éléments chimiques inorganiques contenus dans ledit échantillon,
   l'étape a) étant réalisé sur *n* surfaces différentes de l'échantillon pour déterminer *n* concentrations massiques de chacun des éléments chimiques inorganiques contenus dans ledit échantillon, avec *n* un nombre entier supérieur ou égal à 2, en particulier compris entre 2 et 20, tout particulièrement entre 5 et 15, encore plus particulièrement égal à 10,
b) calcul de la moyenne des *n* concentrations massiques de chacun des éléments chimiques inorganiques contenus dans ledit échantillon et de l'écart type de cette moyenne,
c) classification de chacun des éléments chimiques inorganiques contenus dans le matériau excavé selon les critères suivants :
   - l'éléments chimique inorganique dont la moyenne des *n* concentrations massiques dans le matériau excavé est supérieure à 1% et dont l'écart type de cette moyenne est inférieur à 40% est classé comme élément chimique inorganique majeur homogène,
   - l'éléments chimique inorganique dont la moyenne des *n* concentrations massiques dans le matériau excavé est supérieure à 1% et dont l'écart type de cette moyenne est supérieur à 40% est classé comme élément chimique inorganique majeur dispersé,
   - l'éléments chimique inorganique dont la moyenne des *n* concentrations massiques dans le matériau excavé est comprise entre 0,01 % et 1% et dont l'écart type de cette moyenne est inférieur à 40% est classé comme élément chimique inorganique mineur homogène,
   - l'éléments chimique inorganique dont la moyenne des *n* concentrations massiques dans le matériau excavé est comprise entre 0,01 % et 1% et dont l'écart type de cette moyenne est supérieur à 40% est classé comme élément chimique inorganique mineur dispersé,
   - l'éléments chimique inorganique dont la moyenne des *n* concentrations massiques dans le matériau excavé est inférieure à 0,01% et dont l'écart type de cette moyenne est inférieure à 40% est classé comme élément chimique inorganique trace homogène, ou
   - l'éléments chimique inorganique dont la moyenne des *n* concentrations massiques dans le matériau excavé est inférieure à 0,01% et dont l'écart type de cette moyenne est supérieure à 40% est classé comme élément chimique inorganique trace dispersé, et
d) identification de la couche lithologique du matériau excavé à partir de la classification réalisée au cours de l'étape c).

Au sens de la présente demande, "matériau excavé" (aussi appelé "matériau d'excavation") désigne tout matériau issu de travaux de génie civil ou de construction que ce soit à la surface de la Terre, par exemple lors de fouilles ou de création de fondation, ou dans le sous-sol, par exemple lors du creusement de tunnels, cavernes et galeries. Le matériau excavé comprend, par exemple, des :
- roches meubles tels que graviers, sables, limons, argiles ou leurs mélanges;
- rochers concassés;
- matériaux provenant de constructions antérieures ou de sites pollués tels que des décharges; ou
- boues.

Par exemple, le matériau excavé est un matériau extrait par un tunnelier utilisé pour la construction d'une ligne de métro, d'une ligne de train, d'une route.

Au sens de la présente demande, "éléments chimiques inorganiques" désigne les éléments chimiques inorganiques des groupes des métaux alcalins, des alcalino-terreux, des lanthanides, des actinides, des métaux de transitions, des métaux pauvres, des métalloïdes, des non-métaux et des halogènes, en particulier des groupes des alcalino-terreux, des métaux de transition, des métaux pauvres, des métalloïdes, des non-métaux et des halogènes. Typiquement, les éléments chimiques inorganiques peuvent être choisis parmi Ag, AI, As, Ba, Bi, Ca, Cd, CI, Co, Cr, Cu, F, Fe, Hg, K, Mg, Mn, Mo, Ni, Nb, P, Pb, Rb, S, Sb, Se, Si, Sn, Sr, Ti, V, Zn et Zr, en particulier choisi parmi Ag, AI, As, Ba, Bi, Ca, Cd, CI, Co, Cr, Cu, Fe, K, Mg, Mn, Mo, Ni, Nb, P, Pb, Rb, S, Sb, Si, Sn Sr, Ti, V, Zn et Zr.

Au sens de la présente demande, "spectroscopie par fluorescence aux rayons X" (aussi appelée "spectrométrie par fluorescence des rayons X" ou "X-ray fluorescence spectroscopy" selon la terminologie anglaise) désigne une technique d'analyse chimique utilisant une propriété physique de la matière, la fluorescence aux rayons X. Les atomes possèdent tous un nombre d'électrons définis, et chacun de ces électrons a une énergie propre définie par son niveau atomique. Les niveaux atomiques correspondent à une énergie et peuvent contenir un nombre d'électrons précis. Plus un niveau d'énergie est haut en énergie, plus il y a de place mais moins les électrons auront tendance à le remplir. Un électron peut être excité par un photon et changer de niveau d'énergie, passant à un niveau d'énergie plus élevée. La place laissée dans le niveau d'énergie par l'électron est alors comblée par un électron d'un autre niveau d'énergie. La transition de niveau d'énergie libère de l'énergie, sous différentes formes. Le phénomène qui nous intéresse pour la fluorescence aux rayons X est l'émission d'un photon de fluorescence dans le domaine des rayons X. L'énergie de chaque photon émis de la sorte est précisément connue pour chaque élément et les différentes transitions possibles pour un élément sont décrites. Il existe ainsi des tables donnant pour chaque élément toutes les énergies d'émission possible et l'énergie minimale pour exciter l'atome. Le spectre obtenu par la spectroscopie par fluorescence aux rayons X est caractéristique de l'échantillon analysé.

Selon un mode de réalisation, l'étape a) d'analyse peut être réalisée directement sur la surface du matériau excavé, l'échantillon étant alors le matériau excavé, ou sur la surface d'un prélèvement du matériau excavé, l'échantillon étant alors le prélèvement.

Selon un mode de réalisation, l'étape a) d'analyse par spectroscopie par fluorescence aux rayons X peut être réalisée avec une énergie variant de 0 à 100 keV, en particulier de 0 à 50 keV, tout particulièrement de 0 à 20 keV.

Selon un mode de réalisation particulier, l'étape a) d'analyse par spectroscopie par fluorescence aux rayons X peut être réalisée sur une ou plusieurs plages d'énergie, chaque plage d'énergie allant de -5 keV, en particulier de -3 keV, tout particulièrement de -2 keV d'une énergie centrale à +5 keV, en particulier à +3 keV, tout particulièrement à +2 keV de l'énergie centrale, l'énergie centrale étant l'énergie d'une raie caractéristique d'un élément chimique inorganique contenu dans l'échantillon analysé.

De façon avantageuse, réaliser l'analyse par spectroscopie par fluorescence aux rayons X sur différentes plages d'énergie permet d'augmenter le signal reçu et donc d'augmenter le rapport signal/bruit. En conséquence, les éléments chimiques inorganiques, en particulier les éléments polluants inorganiques contenus dans l'échantillon analysé sont plus facilement détectés et plus facilement dosés.

Des tables donnant l'énergie centrale pour chaque élément chimique inorganique, en particulier chaque élément polluant inorganique, sont connues de l'homme du métier.

Selon un mode de réalisation, le temps total d'acquisition lors de l'étape a) d'analyse par spectroscopie par fluorescence aux rayons X peut être de 0,25 min à 10 min, en particulier de 0,5 min à 5 min, tout particulièrement est de 1 min à 3 min.

De façon avantageuse, un temps total d'acquisition dans ces plages de valeurs permet de recevoir un signal de qualité et donc d'améliorer la fiabilité de la détermination de la concentration massique de chacun des éléments chimiques inorganiques contenus dans l'échantillon analysé.

De plus la durée des *n* étapes a) d'analyse par spectroscopie par fluorescence aux rayons X est avantageusement très inférieure à celle de l'analyse classique par diffraction aux rayons X.

Selon un mode de réalisation, l'étape a) d'analyse par spectroscopie par fluorescence aux rayons X peut être réalisée avec une tension d'accélération des électrons comprise entre 5 kV et 200 kV, en particulier entre 25 kV et 100 kV, plus particulier une tension d'accélération de 50 kV.

De façon avantageuse, une valeur de tension d'accélération dans ces plages permet d'exciter à la fois les éléments chimiques inorganiques les plus légers et les éléments chimiques inorganiques, en particulier les éléments polluants les plus lourds, contenus dans l'échantillon analysé. Cela permet donc de déterminer la concentration massique de chacun des éléments chimiques inorganiques, en particulier les éléments polluants contenus dans l'échantillon analysé.

De façon avantageuse, la spectroscopie par fluorescence aux rayons X permet de déterminer la concentration massique de chaque élément de l'échantillon en moins de dix minutes, de façon non destructive et sans étape préalable de préparation d'échantillon telle qu'un séchage, un broyage éventuellement suivi d'un tamisage et un pastillage.

Ainsi, selon un mode de réalisation particulier, le procédé de l'invention ne comprend pas d'étape de préparation de l'échantillon du matériau excavé, en particulier d'étape de préparation choisie parmi :
- une étape de séchage de l'échantillon ;
- une étape de broyage de l'échantillon suivi, éventuellement d'une étape de tamisage ; et
- une étape de pastillage de l'échantillon.

La moyenne des n concentrations massiques dans le matériau excavé de chaque élément chimique inorganique contenu dans le matériau excavé permet de classer ledit matériau excavé comme élément chimique inorganique majeur, élément chimique inorganique mineur ou élément chimique inorganique trace.

Typiquement, un élément chimique inorganique :
- dont la moyenne des *n* concentrations massiques dans le matériau excavé est supérieure à 1% peut être classé comme élément chimique inorganique majeur,
- dont la moyenne des *n* concentrations massiques dans le matériau excavé est comprise entre 0,01% et 1% peut être classé comme élément chimique inorganique mineur, ou
- dont la moyenne des *n* concentrations massiques dans le matériau excavé est inférieure à 0,01% peut être classé comme élément chimique inorganique trace.

Par exemple, en fonction du matériau excavé, AI, Ba, Ca, Fe, K, Mg, S et Si peuvent être classés comme un élément chimique inorganique majeur, As, CI, Cr, Cu, Mn, Ni, P, Rb, Sr, Ti, V, Zn et Zr peuvent être classés comme un élément chimique inorganique mineur et Ag, Bi, Cd, Co, Mo, Nb, Pb, Sb et Sn peuvent être classés comme un élément chimique inorganique trace.

L'écart type de la moyenne des *n* concentrations massiques dans le matériau excavé de chaque élément chimique inorganique contenu dans le matériau excavé permet de classer ledit matériau excavé en fonction de sa dispersibilité, *i.e.* homogène ou dispersé.

Typiquement, un élément chimique inorganique :
- dont l'écart type de la moyenne des *n* concentrations massiques dans le matériau excavé est inférieur à 40% peut être classé comme élément chimique inorganique homogène, ou
- dont l'écart type de la moyenne des *n* concentrations massiques dans le matériau excavé est supérieure à 40% peut être classé comme élément chimique inorganique dispersé.

La répartition de chaque élément chimique inorganique contenu dans le matériau excavé en tant qu'élément chimique inorganique majeur, mineur ou trace, permet de pré-identifier les couches lithologiques en fonction de leur composition chimique.
Puis la dispersibilité de chaque élément chimique inorganique contenu dans le matériau excavé permet d'identifier la couche lithologique parmi les couches lithologiques pré-identifiées. Cette identification est réalisée grâce à la structure de la couche lithologique. En effet une couche lithologique présente une structure spécifique en fonction de la dispersibilité de tout ou partie des éléments chimiques inorganiques contenus dans ladite couche lithologique.

Par exemple, les éléments majeurs Ba, Fe, K, AI, Si et Mg ; les éléments mineurs P, Ti, V, Cr, Ni et Zn et les éléments traces Pb et Bi permettent de pré-identifier les couches lithologiques Gneiss du Sapey GS et Micaschistes ξζ. Si l'élément majeur Mg est homogène, alors la couche lithologique est Gneiss du Sapey GS. Si l'élément Mg est dispersé alors la couche lithologique est Micaschistes ξζ.

Par exemple, les éléments majeurs Fe, Ti, Ca, K, AI, Si et Mg, les éléments mineurs Ba, Zr, Rb, Cr, V et P, et les éléments traces Nb, Bi et Pb permettent de pré-identifier les couches lithologiques schistes gréseux ou schistes micacés (phyllosilicates). Si l'élément trace Bi est homogène, alors la couche lithologique est schistes gréseux. Si l'élément trace Bi est dispersé alors la couche lithologique est schistes micacés (phyllosilicates).

La pré-identification et l'identification peuvent être réalisées à l'aide de cartes géologiques et leur notice géologique associée ou à l'aide de livres de géologie connus de l'homme du métier tels que *"Détermination des minéraux des roches au microscope polarisant"* de Marcel Roubault (édition Lamarre-Poinat).

De façon avantageuse, la classification de chaque élément chimique inorganique contenu dans le matériau excavé grâce à la moyenne des n concentrations massiques et à l'écart type de cette moyenne permet d'identifier la couche lithologique d'un matériau excavé de façon fiable.

Chaque couche lithologique présente des propriétés géomécaniques et chimiques spécifiques connues. Leur identification par le procédé de l'invention permet donc de conclure de façon fiable, rapide et en accord avec les recommandations actuelles si le matériau excavé peut être valorisé en tant que matériau de construction et en quel matériau de construction il peut être valorisé, par exemple en tant que remblai ou granulat.

Ainsi, selon un autre aspect, la présente divulgation porte également sur un procédé de valorisation d'un matériau excavé mettant en oeuvre le procédé d'identification tel que défini ci-dessus et comprenant en outre l'étape suivante : e) valorisation du matériau excavé en tant que matériau de construction en fonction de l'identification obtenue lors de l'étape d).

Le matériau excavé peut être valorisé en tant que matériau de construction si sa couche lithologique lui confère des propriétés géomécaniques et chimiques adaptées à cette valorisation. Par exemple, ces propriétés géomécaniques et chimiques peuvent être :
une teneur moyenne en soufre inférieure ou égale à 0,45%,
une valeur moyenne de classification des masses rocheuses ("Rock Mass Rating", RMR, selon la terminologie anglaise) supérieure ou égale à 40, et
une valeur minimale de résistance à la compression uniaxale ("Unconfined Compressive Strength", UCS, selon la terminologie anglaise) supérieure ou égale à 2 MPa.

La teneur moyenne en soufre peut être déterminée par le procédé d'analyse de la présente invention. La valeur moyenne de classification des masses rocheuses peut être déterminée selon le système RMR89. La valeur minimale de résistance à la compression uniaxale peut être déterminée selon la norme NF P94-420 (2000).

Un matériau excavé dont la couche lithologique lui confère une de ces propriétés géomécaniques et chimiques peut subir l'étape e) de valorisation. Par exemple, les couches lithologiques calcaire marneux jmCM, calcaire tCd, dolomie tBD, grès hBo, micaschiste AMD, micaschiste AMF, micaschiste de l'Arpont Sv, quartzite rt, quartzite tQ, quarztite QSE et schiste micacé confèrent une de ces propriétés géomécaniques et chimiques au matériau excavé.

Ainsi un matériau excavé dont la couche lithologique est calcaire tCd, dolomie tBD, grès hBo, micaschiste AMD, micaschiste AMF, calcaire marneux jmCM, quartzite rt, quartzite tQ, quarztite QSE et schiste micacé est adapté à une valorisation en tant que matériau de construction. Il peut donc subir l'étape e) de valorisation du procédé de valorisation de l'invention.

Les propriétés géomécaniques et chimiques conférées par la couche lithologique au matériau excavé sont connues. L'homme du métier peut par exemple trouver cette information dans les mémoires d'étude géotechniques traitant de la géomécanique de sols et dans les bases de données publiées par le Bureau de Recherches Géologiques et Minières (BRGM) français.

Typiquement, le matériau de construction peut être un remblai ou un granulat, en particulier un remblai, un granulat pour béton ou un granulat pour enrobé.

Au sens de la présente demande, "remblai" désigne un matériau de construction destiné à élever un terrain, combler un creux ou combler les vides de l'exploitation minière.

Au sens de la présente demande, "granulat" désigne un matériau de construction utilisé pour la réalisation d'ouvrages de Génie Civil, de travaux routiers et de bâtiments.

Un granulat pour béton et un granulat pour enrobé sont des exemples de granulat.

Au sens de la présente demande, "granulat pour enrobé" désigne un granulat utilisé pour la confection du bitume.

Au sens de la présente demande, "granulat pour béton" désigne un granulat utilisé pour la confection des bétons.

L'étape e) de valorisation peut comprendre une étape de production d'un matériau de construction à partir du matériau d'excavation.

Typiquement l'étape de production peut comprendre une ou des sous-étapes de mise en forme du matériau excavé, la ou les sous-étapes étant adaptées au matériau de construction, en particulier au remblai ou au granulat, plus particulièrement au remblai, au granulat pour béton ou au granulat pour enrobé.

Un matériau excavé dont la couche lithologique lui confère les propriétés géomécaniques et chimiques suivantes :
une teneur moyenne en soufre inférieure ou égale à 0,45%,
une valeur moyenne de classification des masses rocheuses supérieure ou égale à 40, et
une valeur minimale de résistance à la compression uniaxale supérieure ou égale à 2 Mpa,
est adapté à une valorisation en tant que remblai. Ce matériau peut donc subir une ou des sous-étapes de mise en forme adapté aux remblais.

Typiquement les couches lithologiques calcaire marneux jmCM, calcaire tCd, dolomie tBD, grès hBo, micaschiste AMD, micaschiste AMF, quartzite rt, quartzite tQ, quarztite QSE et schiste micacé, en particulier micaschiste AMD confèrent les propriétés mentionnées ci-dessus au matériau excavé.

Ainsi un matériau excavé dont la couche lithologique peut être micaschiste AMD, micaschiste AMF, calcaire marneux jmCM, schiste micacé ou leurs mélanges, en particulier micaschiste AMD est adapté à une valorisation en tant que remblai. Il peut donc subir une ou des sous-étapes de mise en forme adapté aux remblais.

Un matériau excavé dont la couche lithologique lui confère les propriétés géomécaniques et chimiques suivantes :
une teneur moyenne en soufre inférieure ou égale à 0,45%,
une valeur moyenne de classification des masses rocheuses supérieure ou égale à 40, et
une valeur minimale de résistance à la compression uniaxale supérieure à 2 Mpa, est adapté à une valorisation en tant que granulat. Ce matériau excavé peut donc subir une ou des étapes de mise en forme adapté aux granulats.

Typiquement les couches lithologiques calcaire tCd, dolomie tBD, grès hBo, micaschiste AMD, micaschiste AMF, micaschiste de l'Arpont Sv quartzite rt, quartzite tQ, quarztite QSE, en particulier quartzite rt et quartzite QSE confèrent les propriétés mentionnées ci-dessus au matériau excavé.

Ainsi un matériau excavé dont la couche lithologique peut être calcaire tCd, dolomie tBD, grès hBo, micaschiste AMD, micaschiste AMF, micaschiste de l'Arpont Sv, quartzite rt, quartzite tQ et quarztite QSE ou leurs mélanges en particulier quartzite rt, quartzite QSE ou leur mélange est adapté à une valorisation en tant que granulat. Il peut donc subir une ou des sous-étapes de mise en forme adapté aux granulats.

Un matériau excavé dont la couche lithologique lui confère les propriétés géomécaniques et chimiques suivantes :
une teneur moyenne en soufre inférieure ou égale à 0,45%,
une valeur moyenne de classification des masses rocheuses supérieure ou égale à 40, et
une valeur minimale de résistance à la compression uniaxale supérieure à 2 MPa et inférieure ou égale à 37 Mpa est adapté à une valorisation en tant que granulat pour enrobé. Ce matériau excavé peut donc subir une ou des étapes de mise en forme adapté aux granulats pour enrobé.

Typiquement les couches lithologiques calcaire tCd, dolomie tBD, quartzite rt, quartzite tQ, quarztite QSE, en particulier quartzite rt et quartzite QSE confèrent les propriétés mentionnées ci-dessus au matériau excavé.

Ainsi un matériau excavé dont la couche lithologique est calcaire tCd, dolomie tBD, quartzite rt, quartzite tQ, quarztite QSE ou leurs mélanges en particulier quartzite rt, quartzite QSE ou leur mélange est adapté à une valorisation en tant que granulat pour enrobé. Il peut donc subir une ou des sous-étapes de mise en forme adapté aux granulats pour enrobé.

Un matériau excavé dont la couche lithologique lui confère les propriétés géomécaniques et chimiques suivantes :
une teneur moyenne en soufre inférieure à 0,25%,
une valeur moyenne de classification des masses rocheuses supérieure ou égale à 40, et
une valeur minimale de résistance à la compression uniaxale supérieure à 2 MPa. est adapté à une valorisation en tant que granulat pour béton. Ce matériau excavé peut donc subir une ou des étapes de mise en forme adapté aux granulats pour béton.

Typiquement les couches lithologiques calcaire tCd, dolomie tBD, grès hBo, micaschiste AMD, micaschiste AMF, micaschiste de l'Arpont Sv, quarztite QSE et quartzite tQ, en particulier, calcaire tCd, dolomie tBD, quartzite QSE et quartzite tQ, et tout particulièrement quartzite tQ confèrent les propriétés mentionnées ci-dessus au matériau excavé.

Ainsi un matériau excavé dont la couche lithologique peut être calcaire tCd, dolomie tBD, grès hBo, micaschiste AMD, micaschiste AMF, micaschiste de l'Arpont Sv, quarztite QSE et quartzite tQ, en particulier, calcaire tCd, dolomie tBD, quartzite QSE et quartzite tQ, et tout particulièrement quartzite tQ est adapté à une valorisation en tant que granulat pour béton. Il peut donc subir une ou des sous-étapes de mise en forme adapté aux granulats pour béton.

La ou les sous-étapes de mise en forme du procédé de production selon l'invention sont des étapes classiques que l'homme du métier saura adapter au matériau excavé et au matériau de construction produit.

Selon un autre aspect, la présente divulgation porte également sur un procédé d'analyse d'un matériau excavé comprenant les étapes a) et b) telles que définies ci-dessus.

Selon un autre aspect, la présente divulgation porte également sur un procédé de valorisation d'un matériau excavé mettant en oeuvre le procédé d'analyse tel que défini ci-dessus et comprenant en outre les étape c) et d) telles que définies ci-dessus et l'étape e) de valorisation telle que définie ci-dessus.

L'invention va être décrite plus en détail à l'aide des exemples suivants donnés à titre d'illustration seulement.

### Exemples

### Exemple 1 : Analyse d'un premier matériau excavé selon le procédé de l'invention

Dix surfaces différentes d'un échantillon de matériau excavé lors du creusement d'un tunnel sur le tracé de la ligne Lyon-Turin en Vallée de Maurienne ont été analysées par spectroscopie par fluorescence aux rayons X. Aucune étape de préparation de l'échantillon n'a été réalisée avant l'analyse.

La moyenne des dix concentrations massiques de chacun des éléments chimiques inorganiques contenus dans cet échantillon de matériau excavé et l'écart type de cette moyenne ont été déterminés.

Les résultats sont présentés dans le Tableau 1 ci-dessous.

**[Tableau 1]**

| Elément inorganique | Moyenne des dix concentrations massiques | Ecart type | |
|---|---|---|---|
| | (%) | (%) | |
| Ba | 0,01 | 37,5 | Elément mineur homogène |
| Zr | 0,004 | 64,22 | Elément trace dispersé |
| Sr | 0,00005 | 301,51 | Elément trace dispersé |
| Rb | 0,003 | 35,92 | Elément trace homogène |
| Bi | 0,0002 | 301,51 | Elément trace dispersé |
| As | 0,00006 | 301,51 | Elément trace dispersé |
| Fe | 0,39 | 42,09 | Elément mineur dispersé |
| Mn | 0,003 | 301,51 | Elément trace dispersé |
| Cr | 0,003 | 108,39 | Elément trace dispersé |
| Ti | 0,05 | 47,13 | Elément mineur dispersé |
| Ca | 0,06 | 94,46 | Elément mineur dispersé |
| K | 2,16 | 42,71 | Elément majeur dispersé |
| Al | 2,82 | 49,4 | Elément majeur dispersé |
| Si | 34,44 | 31,8 | Elément majeur homogène |
| Cl | 0,01 | 301,51 | Elément mineur dispersé |
| S | 0,02 | 181,76 | Elément mineur dispersé |
| Mg | 0,09 | 204,95 | Elément mineur dispersé |

La couche lithologique du matériau excavé est Quartzite QSE. Ceci est en accord avec une analyse par diffraction des rayons X menées sur un autre échantillon du même matériau excavé. 30 minutes se sont écoulées entre la production du matériau excavé et le résultat de l'analyse par spectroscopie par fluorescence aux rayons X. Par contre plus de 5 jours se sont écoulées entre la production du matériau excavé et le résultat de l'analyse par diffraction des rayons X.

Ce matériau excavé peut être valorisé en tant que remblais ou granulats.

### Exemple 2 : Analyse d'un deuxième matériau excavé selon le procédé de l'invention

Dix surfaces différentes d'un échantillon de matériau excavé lors du creusement d'un tunnel sur le tracé de la ligne Lyon-Turin en Vallée de Maurienne ont été analysées par spectroscopie par fluorescence aux rayons X. Aucune étape de préparation de l'échantillon n'a été réalisée avant l'analyse.

La moyenne des dix concentrations massiques de chacun des éléments chimiques inorganiques contenus dans cet échantillon de matériau excavé et l'écart type de cette moyenne ont été déterminés.

Les résultats sont présentés dans le Tableau 2 ci-dessous.

La couche lithologique du matériau excavé est micaschiste de l'Arpont Sv. Ceci est en accord avec une analyse par diffraction des rayons X menées sur un autre échantillon du même matériau excavé. 30 minutes ce sont écoulées entre la production du matériau excavé et le résultat de l'analyse par spectroscopie par fluorescence aux rayons X. Par contre plus de 5 jours se sont écoulées entre la production du matériau excavé et le résultat de l'analyse par diffraction des rayons X.

Ce matériau excavé peut être valorisé en tant que granulats pour béton.

### Exemple 3 : Analyse d'un troisième matériau excavé selon le procédé de l'invention

Dix zones différentes d'un échantillon de matériau excavé lors du creusement d'un tunnel sur le tracé de la ligne Lyon-Turin en Vallée de Maurienne ont été analysées par spectroscopie par fluorescence aux rayons X. Aucune étape de préparation de l'échantillon n'a été réalisée avant l'analyse.

La moyenne des dix concentrations massiques de chacun des éléments chimiques inorganiques contenus dans cet échantillon de matériau excavé et l'écart type de cette moyenne ont été déterminés.

Les résultats sont présentés dans le Tableau 3 ci-dessous.

La couche lithologique du matériau excavé est Calcaire marneux jmCM. Ceci est en accord avec une analyse par diffraction des rayons X menées sur un autre échantillon du même matériau excavé. 30 minutes ce sont écoulées entre la production du matériau excavé et le résultat de l'analyse par spectroscopie par fluorescence aux rayons X. Par contre plus de 5 jours se sont écoulées entre la production du matériau excavé et le résultat de l'analyse par diffraction des rayons X.

Ce matériau excavé peut être valorisé en tant que remblai.

**[Tableau 2]**

| Elément inorganique | Moyenne des dix concentrations massiques (%) | Ecart type (%) | |
|---|---|---|---|
| Ba | 0,15 | 42,86 | Elément mineur dispersé |
| Mo | 0,00004 | 316,23 | Elément trace dispersé |
| Nb | 0,002 | 13,93 | Elément trace homogène |
| Zr | 0,03 | 8,73 | Elément mineur homogène |
| Sr | 0,01 | 50,29 | Elément mineur dispersé |
| Rb | 0,02 | 9,83 | Elément mineur homogène |
| Bi | 0,001 | 40,17 | Elément trace dispersé |
| As | 0,0006 | 116,3 | Elément trace dispersé |
| Pb | 0,0005 | 93,07 | Elément trace dispersé |
| Zn | 0,002 | 59,05 | Elément trace dispersé |
| Cu | 0,004 | 55,45 | Elément trace dispersé |
| Fe | 2,05 | 33,89 | Elément majeur homogène |
| Mn | 0,02 | 132,33 | Elément mineur dispersé |
| Cr | 0,01 | 33,54 | Elément mineur homogène |
| V | 0,01 | 24,79 | Elément mineur homogène |
| Ti | 0,66 | 31,1 | Elément mineur homogène |
| Ca | 0,25 | 71,82 | Elément mineur dispersé |
| K | 4,2 | 23,49 | Elément majeur homogène |
| Al | 9,63 | 30,40 | Elément majeur homogène |
| P | 0,02 | 97,15 | Elément mineur dispersé |
| Si | 20,85 | 29,7 | Elément majeur homogène |
| S | 0,05 | 70,57 | Elément mineur dispersé |
| Mg | 0,44 | 109,79 | Elément mineur dispersé |

**[Tableau 3]**

| Elément inorganique | Moyenne des dix concentrations massiques (%) | Ecart type (%) | |
|---|---|---|---|
| Ba | 0,03 | 19,34 | Elément mineur homogène |
| Mo | 0,00003 | 316,23 | Elément trace dispersé |
| Nb | 0,0001 | 133,36 | Elément trace dispersé |
| Zr | 0,0001 | 87,71 | Elément trace dispersé |
| Sr | 0,19 | 38,36 | Elément mineur homogène |
| Rb | 0,0002 | 106,98 | Elément trace dispersé |
| As | 0,0002 | 131,71 | Elément trace dispersé |
| Pb | 0,0007 | 109,49 | Elément trace dispersé |
| Zn | 0,01 | 106,6 | Elément mineur dispersé |
| Cu | 0,0009 | 169,89 | Elément trace dispersé |
| Ni | 0,0008 | 316,23 | Elément trace dispersé |
| Fe | 0,37 | 60,67 | Elément mineur dispersé |
| Mn | 0,01 | 69,77 | Elément mineur dispersé |
| Cr | 0,01 | 46,22 | Elément mineur dispersé |
| V | 0,0002 | 316,23 | Elément trace dispersé |
| Ti | 0,09 | 37,44 | Elément mineur homogène |
| Ca | 35 | 16,98 | Elément majeur homogène |
| K | 0,21 | 69,14 | Elément mineur dispersé |
| Al | 1,19 | 53 | Elément majeur dispersé |
| P | 0,02 | 75,01 | Elément mineur dispersé |
| Si | 7,71 | 43,40 | Elément majeur dispersé |
| Cl | 0,03 | 59,79 | Elément mineur dispersé |
| S | 0,17 | 26,27 | Elément mineur homogène |
| Mg | 0,10 | 316,23 | Elément mineur dispersé |

## Revendications

1. Procédé d'identification de la couche lithologique d'un matériau excavé comprenant les étapes suivantes :
a) analyse par spectroscopie par fluorescence aux rayons X d'un échantillon du matériau excavé pour déterminer la concentration massique de chacun des éléments chimiques inorganiques contenus dans ledit échantillon,
l'étape a) étant réalisé sur *n* surfaces différentes de l'échantillon pour déterminer *n* concentrations massiques de chacun des éléments chimiques inorganiques contenus dans ledit échantillon, avec *n* un nombre entier supérieur ou égal à 2,
b) calcul de la moyenne des *n* concentrations massiques de chacun des éléments chimiques inorganiques contenus dans ledit échantillon et de l'écart type de cette moyenne,
le procédé étant **caractérisé en ce qu'**il comprend aussi les étapes suivantes:
c) classification de chacun des éléments chimiques inorganiques contenus dans le matériau excavé selon les critères suivants :
- l'éléments chimique inorganique dont la moyenne des *n* concentrations massiques dans le matériau excavé est supérieure à 1% et dont l'écart type de cette moyenne est inférieur à 40% est classé comme élément chimique inorganique majeur homogène,
- l'éléments chimique inorganique dont la moyenne des *n* concentrations massiques dans le matériau excavé est supérieure à 1% et dont l'écart type de cette moyenne est supérieur à 40% est classé comme élément chimique inorganique majeur dispersé,
- l'éléments chimique inorganique dont la moyenne des *n* concentrations massiques dans le matériau excavé est comprise entre 0,01 % et 1% et dont l'écart type de cette moyenne est inférieur à 40% est classé comme élément chimique inorganique mineur homogène,
- l'éléments chimique inorganique dont la moyenne des *n* concentrations massiques dans le matériau excavé est comprise entre 0,01 % et 1% et dont l'écart type de cette moyenne est supérieur à 40% est classé comme élément chimique inorganique mineur dispersé,
- l'éléments chimique inorganique dont la moyenne des *n* concentrations massiques dans le matériau excavé est inférieure à 0,01% et dont l'écart type de cette moyenne est inférieure à 40% est classé comme élément chimique inorganique trace homogène, ou
- l'éléments chimique inorganique dont la moyenne des *n* concentrations massiques dans le matériau excavé est inférieure à 0,01% et dont l'écart type de cette moyenne est supérieure à 40% est classé comme élément chimique inorganique trace dispersé, et
d) identification de la couche lithologique du matériau excavé à partir de la classification réalisée au cours de l'étape c).

2. Procédé selon la revendication 1 dans lequel le matériau excavé est un matériau extrait par un tunnelier utilisé pour la construction d'une ligne de métro, d'une ligne de train, d'une route.

3. Procédé selon la revendication 1 ou la revendication 2 dans lequel les éléments chimiques inorganiques sont choisis parmi Ag, AI, As, Ba, Bi, Ca, Cd, CI, Co, Cr, Cu, F, Fe, Hg, K, Mg, Mn, Mo, Ni, Nb, P, Pb, Rb, S, Sb, Se, Si, Sn, Sr, Ti, V, Zn et Zr.

4. Procédé selon l'une quelconque des revendications 1 à 3 ne comprenant pas d'étape de préparation de l'échantillon du matériau excavé.

5. Procédé de valorisation d'un matériau excavé mettant en oeuvre le procédé d'identification tel que défini dans l'une quelconque des revendication 1 à 4, comprenant en outre l'étape suivante :
e) valorisation du matériau excavé en tant que matériau de construction en fonction de l'identification obtenue lors de l'étape d).

6. Procédé selon la revendication 5 dans lequel le matériau de construction est un remblai ou un granulat.

7. Procédé selon la revendication 5 ou la revendication 6 dans lequel l'étape e) de valorisation comprend une étape de production d'un matériau de construction à partir du matériau d'excavation.

8. Procédé selon la revendication 7 dans lequel l'étape de production comprend une ou des sous-étapes de mise en forme du matériau excavé, la ou les sous-étapes étant adaptées au matériau de construction.

9. Procédé selon la revendication 8 dans lequel le matériau excavé dont la couche lithologique lui confère les propriétés géomécaniques et chimiques suivantes :
une teneur moyenne en soufre inférieure ou égale à 0,45%,
une valeur moyenne de classification des masses rocheuses supérieure ou égale à 40, et
une valeur minimale de résistance à la compression uniaxale supérieure ou égale à 2 Mpa,
subit une ou des sous-étapes de mise en forme adapté aux remblais.

10. Procédé selon la revendication 9 dans lequel la couche lithologique du matériau excavé est micaschiste AMD, micaschiste AMF, calcaire marneux jmCM, schiste micacé ou leurs mélanges.

11. Procédé selon la revendication 8 dans lequel le matériau excavé dont la couche lithologique lui confère les propriétés géomécaniques et chimiques suivantes :
une teneur moyenne en soufre inférieure ou égale à 0,45%,
une valeur moyenne de classification des masses rocheuses supérieure ou égale à 40, et
une valeur minimale de résistance à la compression uniaxale supérieure à 2 Mpa, subit une ou des sous-étapes de mise en forme adapté au granulat.

12. Procédé selon la revendication 11 dans lequel la couche lithologique du matériau excavé est calcaire tCd, dolomie tBD, grès hBo, micaschiste AMD, micaschiste AMF, quartzite rt, quartzite tQ et quarztite QSE ou leurs mélanges.

## Patentansprüche

1. Verfahren zur Identifizierung der lithologischen Schicht eines Aushubmaterials, das die folgenden Schritte umfasst:
a) Röntgenfluoreszenzspektroskopie-Analyse einer Probe des Aushubmaterials zur Bestimmung der Massenkonzentration jedes in der Probe enthaltenen anorganischen chemischen Elements,
wobei Schritt a) auf n verschiedenen Oberflächen der Probe durchgeführt wird, um n Massenkonzentrationen jedes in der Probe enthaltenen anorganischen chemischen Elements zu bestimmen, wobei n eine ganze Zahl größer oder gleich 2 ist,
b) Berechnung des Mittelwerts der n Massenkonzentrationen jedes der in der Probe enthaltenen anorganischen chemischen Elemente und der Standardabweichung von diesem Mittelwert, wobei das Verfahren **dadurch gekennzeichnet ist, dass** es auch die folgenden Schritte umfasst:
c) Klassifizierung jedes anorganischen chemischen Elements, das in dem Aushubmaterial enthalten ist, gemäß folgenden Kriterien:
- ein anorganisches chemische Element, bei dem der Mittelwert der n Massenkonzentrationen im Aushubmaterial größer als 1% ist und die Standardabweichung von diesem Mittelwert kleiner als 40 % ist, wird als homogenes anorganisches chemisches Hauptelement klassifiziert,
- ein anorganisches chemisches Element, bei dem der Mittelwert der n Massenkonzentrationen in dem Aushubmaterial größer als 1 % ist und die Standardabweichung von diesem Mittelwert größer als 40 % ist, wird als disperses anorganisches Hauptelement klassifiziert,
- ein anorganisches chemisches Element, bei dem der Mittelwert der n Massenkonzentrationen im Aushubmaterial zwischen 0,01 % und 1 % liegt und die Standardabweichung von diesem Mittelwert kleiner als 40 % ist, wird als homogenes anorganisches chemisches Nebenelement klassifiziert,
- ein anorganisches chemisches Element, bei dem der Mittelwert der n Massenkonzentrationen im Aushubmaterial zwischen 0,01 % und 1 % liegt und die Standardabweichung von diesem Mittelwert größer als 40 % ist, wird als disperses anorganisches chemisches Nebenelement klassifiziert,
- ein anorganisches chemisches Elemente, bei dem der Mittelwert der n Massenkonzentrationen im Aushubmaterial weniger als 0,01 % beträgt und die Standardabweichung von diesem Mittelwert kleiner als 40 % ist, wird als homogenes anorganisches chemisches Nebenelement klassifiziert, oder
- ein anorganisches chemisches Element, bei dem der Mittelwert der n Massenkonzentrationen im Aushubmaterial kleiner als 0,01 % ist und die Standardabweichung von diesem Mittelwert größer als 40 % ist, wird als disperses anorganisches chemisches Nebenelement klassifiziert wird, und
d) Identifizieren der lithologischen Schicht des Aushubmaterials anhand der in Schritt c) vorgenommenen Klassifizierung.

2. Verfahren nach Anspruch 1, wobei es sich bei dem Aushubmaterial um Material handelt, das von einer Tunnelbohrmaschine abgebaut wurde, die zum Bau einer U-Bahn-Linie, einer Zugstrecke oder einer Straße verwendet wird.

3. Verfahren nach Anspruch 1 oder Anspruch 2, wobei die anorganischen chemischen Elemente gewählt sind aus Ag, Al, As, Ba, Bi, Ca, Cd, CI, Co, Cr, Cu, F, Fe, Hg, K, Mg, Mn, Mo, Ni, Nb, P, Pb, Rb, S, Sb, Se, Si, Sn, Sr, Ti, V, Zn und Zr.

4. Verfahren nach einem der Ansprüche 1 bis 3, das keinen Schritt zur Vorbereitung der Probe des Aushubmaterials umfasst.

5. Verfahren zur Verwertung von Aushubmaterial, das den Identifizierungsprozess gemäß einem der Ansprüche 1 bis 4 durchführt und ferner den folgenden Schritt umfasst:
e) Verwerten des Aushubmaterials als Baumaterial auf der Grundlage der in Schritt d) erhaltenen Identifizierung.

6. Verfahren nach Anspruch 5, wobei das Baumaterial eine Aufschüttung oder ein Granulat ist.

7. Verfahren nach Anspruch 5 oder 6, wobei der Verwertungsschritt e) einen Schritt zur Herstellung eines Baumaterials aus dem Aushubmaterial umfasst.

8. Verfahren nach Anspruch 7, wobei der Herstellungsschritt einen Unterschritt oder Unterschritte zur Formgebung des Aushubmaterials umfasst, wobei der Unterschritt oder die Unterschritte an das Baumaterial angepasst sind.

9. Verfahren nach Anspruch 8, wobei das Aushubmaterial, dessen lithologische Schicht ihm die folgenden geomechanischen und chemischen Eigenschaften verleiht:
einen durchschnittlichen Schwefelgehalt von kleiner gleich 0,45 %,
einen durchschnittlichen Wert für die Klassifizierung von Gesteinsmassen von größer gleich 40, und
einen Mindestwert für die einachsige Druckfestigkeit von größer gleich 2 MPa,
einem oder mehreren Unterschritten der Formgebung unterzogen wird, die an die Aufschüttungen angepasst sind.

10. Verfahren nach Anspruch 9, wobei die lithologische Schicht des Aushubmaterials aus AMD-Glimmerschiefer, AMF-Glimmerschiefer, MergelkalkjmCM, Glimmerschiefer oder deren Mischungen besteht.

11. Verfahren nach Anspruch 8, wobei das Aushubmaterial, dessen lithologische Schicht ihm die folgenden geomechanischen und chemischen Eigenschaften verleiht:
einen durchschnittlichen Schwefelgehalt von kleiner gleich 0,45 %,
einen durchschnittlichen Wert für die Klassifizierung von Gesteinsmassen von größer gleich 40, und
einen Mindestwert für die einachsige Druckfestigkeit von größer 2 MPa,
einem oder mehreren Unterschritten der Formgebung unterzogen wird, die an die Gesteinskörnung angepasst ist.

12. Verfahren nach Anspruch 11, wobei die lithologische Schicht des Aushubmaterials Kalkstein tCd, Dolomit tBD, Sandstein hBo, Glimmerschiefer AMD, Glimmerschiefer AMF, Quarzit rt, Quarzit tQ und Quarztit QSE oder deren Mischungen ist.

## Claims

1. Method for identifying the lithological layer of an excavated material, comprising the following steps:
a) analysing a specimen of the excavated material using X-ray fluorescence spectroscopy in order to determine the mass concentration of each of the inorganic chemical elements contained in the said specimen,
step a) being performed on *n* different surfaces of the specimen in order to determine *n* mass concentrations of each of the inorganic chemical elements contained in the said specimen, with *n* being a whole number greater than or equal to 2,
b) calculating the mean of the *n* mass concentrations of each of the inorganic chemical elements contained in the said specimen and the standard deviation of this mean,
the method being **characterized in that** it further comprises the following steps:
c) classifying each of the inorganic chemical elements contained in the excavated material according to the following criteria:
- an inorganic chemical element with a mean of the *n* mass concentrations in the excavated material greater than 1% and a standard deviation of this mean less than 40% is classified as a homogeneous major inorganic chemical element,
- an inorganic chemical element with a mean of the *n* mass concentrations in the excavated material greater than 1% and a standard deviation of this mean greater than 40% is classified as a dispersed major inorganic chemical element,
- an inorganic chemical element with a mean of the *n* mass concentrations in the excavated material which is between 0.01% and 1% and a standard deviation of this mean less than 40% is classified as a homogeneous minor inorganic chemical element,
- an inorganic chemical element with a mean of the *n* mass concentrations in the excavated material which is between 0.01% and 1% and a standard deviation of this mean greater than 40% is classified as a dispersed minor inorganic chemical element,
- an inorganic chemical element with a mean of the *n* mass concentrations in the excavated material less than 0.01% and a standard deviation of this mean less than 40% is classified as a homogeneous trace inorganic chemical element, or
- an inorganic chemical element with a mean of the *n* mass concentrations in the excavated material less than 0.01% and a standard deviation of this mean greater than 40% is classified as a dispersed trace inorganic chemical element, and
d) identifying the lithological layer of the excavated material based on the classification performed during step c).

2. Method according to Claim 1, in which the excavated material is a material extracted by a tunnelling machine used to build an underground rail line, a train line or a road.

3. Method according to Claim 1 or Claim 2, in which the inorganic chemical elements are chosen from Ag, Al, As, Ba, Bi, Ca, Cd, Cl, Co, Cr, Cu, F, Fe, Hg, K, Mg, Mn, Mo, Ni, Nb, P, Pb, Rb, S, Sb, Se, Si, Sn, Sr, Ti, V, Zn and Zr.

4. Method according to any of Claims 1 to 3, not comprising a step in which the specimen of the excavated material is prepared.

5. Method for valorising an excavated material which employs the identification method as defined in any of Claims 1 to 4, furthermore comprising the following step:
e) valorising the excavated material as a building material depending on the identification obtained in step d).

6. Method according to Claim 5, in which the building material is backfill or aggregate.

7. Method according to Claim 5 or Claim 6, in which the valorisation step e) comprises a step of producing a building material from the excavation material.

8. Method according to Claim 7, in which the production step comprises one or more sub-steps of shaping the excavated material, the sub-step or sub-steps being adapted to the building material.

9. Method according to Claim 8, in which the excavated material with a lithological layer which gives it the following geomechanical and chemical properties:
an average sulphur content that is less than or equal to 0.45%,
an average rock mass rating that is greater than or equal to 40, and
a minimum value for the unconfined compressive strength that is greater than or equal to 2 MPa
undergoes one or more sub-steps in which it is shaped suitably for backfill.

10. Method according to Claim 9, in which the lithological layer of the excavated material is mica schist AMD, mica schist AMF, marly limestone jmCM, micaceous schist, or mixtures thereof.

11. Method according to Claim 8, in which in which the excavated material with a lithological layer which gives it the following geomechanical and chemical properties:
an average sulphur content that is less than or equal to 0.45%,
an average rock mass rating that is greater than or equal to 40, and
a minimum value for the unconfined compressive strength that is greater than or equal to 2 MPa
undergoes one or more sub-steps in which it is shaped suitably for aggregate.

12. Method according to Claim 11, in which the lithological layer of the excavated material is limestone tCd, dolomite tBD, sandstone hBo, mica schist AMD, mica schist AMF, quartzite rt, quartzite tQ and quartzite QSE or mixtures thereof.
